# EUROPEAN PATENT SPECIFICATION

(11) **EP 3 502 246 B1**
(45) Date of publication and mention of the grant of the patent: **18.06.2025**
(21) Application number: 17208519.3
(22) Date of filing: 19.12.2017
(51) Int. Cl.: C12N 9/54, C11D 3/386

(54) **AUTOMATIC DISHWASHING DETERGENT COMPOSITION**
SPÜLMITTELZUSAMMENSETZUNG FÜR GESCHIRRSPÜLAUTOMAT
COMPOSITION DE DÉTERGENT DE LAVE-VAISSELLE AUTOMATIQUE

(43) Date of publication of application: 26.06.2019
(73) Proprietor: The Procter & Gamble Company, Cincinnati, OH 45202 (US)
(72) Inventor: JACKSON, Michelle, Newcastle upon Tyne, NE12 9TS (GB); TARBIT, David John, Newcastle-upon-Tyne, NE12 9TS (GB); SOUTER, Philip Frank, Newcastle upon Tyne, NE12 9TS (GB); PEREZ-PRAT de VINUESA, Eva Maria, Newcastle upon Tyne, NE12 9TS (GB); BABE, Lilia Maria, Palo Alto, CA 94304 (US); ESTELL, David Aaron, Palo Alto, CA 94304 (US); GOEDEGEBUUR, Frits, Palo Alto, CA 94304 (US); MULDER, Harm Jan, Palo Alto, CA 94304 (US); PRICELIUS, Sina, Palo Alto, CA 94304 (US); DANKMEYER, Lydia, Palo Alto, CA 94304 (US); KAPER, Thijs, Palo Alto, CA 94304 (US); ENGIN, Hatice Billur, Palo Alto, CA 94304 (US)
(74) Representative: P&G Patent Belgium UK

(56) References cited:
- EP-A1- 2 100 948
- WO-A1-2016/145428
- WO-A1-2016/205755
- WO-A1-2017/192653

## Description

### FIELD OF THE INVENTION

The present invention is in the field of detergents. In particular, it relates to a phosphate-free automatic dishwashing detergent composition comprising a new protease. The composition provides improved cleaning under a plurality of conditions versus compositions comprising conventional proteases.

### BACKGROUND OF INVENTION

There is a permanent desire to improve the performance of automatic dishwashing compositions and their environmental profile.

Due to environmental concerns phosphate is increasingly being replaced by biodegradable complexing agents. These complexing agents can have a strong binding capacity for metals and/or are used in high levels and can negatively affect the performance of enzymes, in particular complexing agents can negatively affect proteases by extracting the structural calcium metal ions of the protease. The proteases can be affected in product and/or in-use. They can be more affected under fully built or overbuilt conditions, i.e., when a composition comprises high level of complexing agent and the composition is used in soft water because there will be more free builder to complex with the structural calcium metal ions of the protease. For the toughest items, consumers would usually select hot, long automatic dishwashing cycles. These cycles create a lot of stress on enzymes.

Automatic dishwashing compositions can be designed to have optimum performance under certain in-use conditions, for example a composition can be designed to have optimum performance in a soft water cycle, however a composition that has optimum performance in soft water might not have optimum performance in a hard water cycle and vice versa.

The object of the present invention is to provide a phosphate-free automatic dishwashing composition that provides better cleaning when used in soft water and preferably under different water hardness's. It is also desirable that the composition provides improved performance even under stressed conditions such as heavily soiled load washed in hot, long cycles.

WO2016/205755A1 relates to variants of *Bacillus gibsonii.*

WO2017/192653A1 relates to a phosphate-free automatic dishwashing cleaning composition comprising an organic complexing agent and a protease.

EP2100948A1 relates to an automatic dishwashing detergent composition comprising an amylase and a protease.

WO2016/145428A1 relates to LG12-clade protease enzymes.

### SUMMARY OF THE INVENTION

According to the first aspect of the present invention, there is provided a phosphate-free automatic dishwashing cleaning composition. The composition comprises a complexing agent system and a novel protease. The composition presents improved cleaning performance on egg and sugary stains such as crème brule. The composition performs really well even when soft water is used in the automatic dishwashing process and even when a hot long cycle is used.

According to the second aspect of the invention there is provided a method of automatic dishwashing using soft water.

The elements of the composition of the invention described in connexion with the first aspect of the invention apply *mutatis mutandis* to the other aspects of the invention.

### DETAILED DESCRIPTION OF THE INVENTION

The present invention encompasses an automatic dishwashing cleaning composition. The composition is phosphate-free and comprises a complexing agent system and a protease. The composition delivers improved cleaning versus cleaning compositions comprising conventional proteases under a plurality of conditions. The composition provides good proteinaceous cleaning, in particular on egg and crème brulee soils. The invention also encompasses methods of automatic dishwashing with soft water and also methods of automatic dishwashing with soft water using hot, long cycles.

By "soft" water is herein meant water having a hardness of less than about 2 gpg (34.3 ppm). Grain per gallon (gpg) is a unit of water hardness defined as 1 grain (64.8 milligrams) of calcium carbonate dissolved in 1 US gallon of water (3.785412 L). It translates into 17.1 parts per million (ppm).

By "hot" cycle is herein understood a dishwashing program in which the main cycle is performed at a temperature above 50°C, preferably above 55°C.

By "long" cycle is herein understood a dishwashing program in which the main cycle has a duration of at least 25, preferably at least 30 and more preferably at least 35 minutes and especially at least 45 minutes.

The composition of the invention comprises a variant protease, the variant proteases have a defined percentage of identity with respect to a reference protease identified by SEQ ID NO 1).

The protease of the composition of the invention is herein sometimes referred to as "the protease of the invention". The proteases having any of sequences ID NO:1 to 10 are herein sometimes referred to as "the reference protease".

The relatedness between two amino acid sequences or between two nucleotide sequences is described by the parameter "sequence identity".

The term "variant" means a protease comprising a mutation, i.e., a substitution, insertion, and/or deletion, at one or more (e.g., several) positions relative to the reference protease. A substitution means replacement of the amino acid occupying a position with a different amino acid; a deletion means removal of the amino acid occupying a position; and an insertion means adding an amino acid adjacent to and immediately following the amino acid occupying a position. The variants of the present invention have at least 90% identity with the reference protease.

The variant presents at least 90%, more preferably at least 92% identity with the protease of SEQ ID NO: 1. SEQ ID NO: 1 corresponds to B. *gibsonii*-clade subtilisin Bgi02446. Also described herein, not part of the invention are variants with at least 90%, more preferably at least 92% identity with the protease of SEQ ID NO: 2. SEQ ID NO: 2 corresponds to B. *lentus* subtilisin. Further described, not part of the invention are variants with at least 90%, more preferably at least 92% identity with one of the proteases of sequences SEQ ID NO: 3-10.

The term "wild-type" protease means a protease expressed by a naturally occurring microorganism, such as a bacterium, yeast, or filamentous fungus found in nature.

### Enzyme related terminology

### Nomenclature for amino acid modifications

In describing enzyme variants herein, the following nomenclature is used for ease of reference: Original amino acid(s): position(s): substituted amino acid(s).

According to this nomenclature, for instance the substitution of glutamic acid for glycine in position 195 is shown as G195E. A deletion of glycine in the same position is shown as G195*, and insertion of an additional amino acid residue such as lysine is shown as G195GK. Where a specific enzyme contains a "deletion" in comparison with other enzyme and an insertion is made in such a position this is indicated as *36D for insertion of an aspartic acid in position 36. Multiple mutations are separated by pluses, i.e.: S99G+V102N, representing mutations in positions 99 and 102 substituting serine and valine for glycine and asparagine, respectively. Where the amino acid in a position (*e.g.* 102) may be substituted by another amino acid selected from a group of amino acids, e.g. the group consisting of N and I, this will be indicated by V102N, I.

In all cases, the accepted IUPAC single letter or triple letter amino acid abbreviation is employed.

### Protease Amino Acid Numbering

The numbering used in this patent is versus the sequences shown and not the BPN' numbering.

### Amino acid identity

The relatedness between two amino acid sequences is described by the parameter "identity". For purposes of the present invention, the alignment of two amino acid sequences is determined by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

The degree of identity between an amino acid sequence of an enzyme used herein ("invention sequence") and a different amino acid sequence ("foreign sequence") is calculated as the number of exact matches in an alignment of the two sequences, divided by the length of the "invention sequence" or the length of the "foreign sequence", whichever is the shortest. The result is expressed in percent identity. An exact match occurs when the "invention sequence" and the "foreign sequence" have identical amino acid residues in the same positions of the overlap. The length of a sequence is the number of amino acid residues in the sequence.

The term "succinate based compound" and "succinic acid based compound" are used interchangeably herein.

As used herein, articles such as "a" and "an" when used in a claim, are understood to mean one or more of what is claimed or described.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated.

### The protease of the invention

The variant has at least 90%, preferably at least 92% identity with the amino acid sequence of SEQ ID NO:1 and comprises the following substitutions: P54T, S126A, D127E and F128G.

The protease of the invention performs very well in phosphate-free compositions even when the compositions are used in soft water.

Preferred levels of protease in the composition of the invention include from about 0.04 to about 5 mg, more preferably from about 0.05 to about 2 mg of active protease per gram of the composition.

### Automatic dishwashing cleaning composition

The automatic dishwashing cleaning composition can be in any physical form. It can be a loose powder, a gel or presented in unit dose form. Preferably it is in unit dose form, unit dose forms include pressed tablets and water-soluble packs. The automatic dishwashing cleaning composition of the invention is preferably presented in unit-dose form and it can be in any physical form including solid, liquid and gel form. The composition of the invention is very well suited to be presented in the form of a multi-compartment pack, more in particular a multi-compartment pack comprising compartments with compositions in different physical forms, for example a compartment comprising a composition in solid form and another compartment comprising a composition in liquid form. The composition is preferably enveloped by a water-soluble film such as polyvinyl alcohol. Especially preferred are compositions in unit dose form wrapped in a polyvinyl alcohol film having a thickness of less than 100 µm, preferably from 20 to 90 µm. The detergent composition of the invention weighs from about 8 to about 25 grams, preferably from about 10 to about 20 grams. This weight range fits comfortably in a dishwasher dispenser. Even though this range amounts to a low amount of detergent, the detergent has been formulated in a way that provides all the benefits mentioned herein above.

The composition is preferably phosphate free. By "phosphate-free" is herein understood that the composition comprises less than 1%, preferably less than 0.1% by weight of the composition of phosphate.

The composition of the invention is phosphate-free and comprises a complexing agent system.

### Complexing agent system

For the purpose of this invention, a "complexing agent" is a compound capable of binding polyvalent ions such as calcium, magnesium, lead, copper, zinc, cadmium, mercury, manganese, iron, aluminium and other cationic polyvalent ions to form a water-soluble complex. The complexing agent has a logarithmic stability constant ([log K]) for Ca2+ of at least 3. The stability constant, log K, is measured in a solution of ionic strength of 0.1, at a temperature of 25° C.

The composition of the invention comprises from 10% to 50% by weight of the composition of a complexing agent system. Preferably, the composition comprises a complexing agent selected from the group consisting of citric acid, methyl glycine diacetic acid (MGDA), glutamic-N,N-diacetic acid (GLDA), iminodisuccinic acid (IDS), carboxy methyl inulin, L-Aspartic acid N, N-diacetic acid tetrasodium salt (ASDA) and mixtures thereof. For the purpose of this invention, the term "acid", when referring to complexing agents, includes acid and salts thereof.

In a preferred embodiment, the composition comprises from 15% to 40% by weight of the invention of MGDA, more preferably the tri-sodium salt of MGDA. Compositions comprising this high level of MGDA perform well in soft water and also in long and/or hot cycles.

In a preferred embodiment, the composition comprises from 15% to 28% by weight of the invention of citric acid, more preferably sodium citrate. Compositions comprising citric acid perform well in soft water.

In a preferred embodiment, the complexing agent system comprises citric acid and MGDA preferably in a weight ratio of from about 0.5:1 to about 5:1, more preferably from about 0.5:1 to about 2.5:1.

### Dispersant polymer

Preferably, the composition of the invention comprises a dispersant polymer. A dispersant polymer can be used in any suitable amount from about 0.1 to about 20%, preferably from 0.2 to about 15%, more preferably from 0.3 to % by weight of the composition.

The dispersant polymer is capable to suspend calcium or calcium carbonate in an automatic dishwashing process.

The dispersant polymer has a calcium binding capacity within the range between 30 to 250 mg of Ca/g of dispersant polymer, preferably between 35 to 200 mg of Ca/g of dispersant polymer, more preferably 40 to 150 mg of Ca/g of dispersant polymer at 25°C. In order to determine if a polymer is a dispersant polymer within the meaning of the invention, the following calcium binding-capacity determination is conducted in accordance with the following instructions:

### Calcium binding capacity test method

The calcium binding capacity referred to herein is determined via titration using a pH/ion meter, such as the Mettler Toledo SevenMulti^{™} bench top meter and a PerfectION^{™} comb Ca combination electrode. To measure the binding capacity a heating and stirring device suitable for beakers or tergotometer pots is set to 25 °C, and the ion electrode with meter are calibrated according to the manufacturer's instructions. The standard concentrations for the electrode calibration should bracket the test concentration and should be measured at 25 °C. A stock solution of 1000 mg/g of Ca is prepared by adding 3.67 g of CaCl₂-2H₂O into 1 L of deionised water, then dilutions are carried out to prepare three working solutions of 100 mL each, respectively comprising 100 mg/g, 10 mg/g, and 1 mg/g concentrations of Calcium. The 100 mg Ca/g working solution is used as the initial concentration during the titration, which is conducted at 25 °C. The ionic strength of each working solution is adjusted by adding 2.5 g/L of NaCl to each. The 100 mL of 100 mg Ca/g working solution is heated and stirred until it reaches 25 °C. The initial reading of Calcium ion concentration is conducted at when the solution reaches 25 °C using the ion electrode. Then the test polymer is added incrementally to the calcium working solution (at 0.01 g/L intervals) and measured after 5 minutes of agitation following each incremental addition. The titration is stopped when the solution reaches 1 mg/g of Calcium. The titration procedure is repeated using the remaining two calcium concentration working solutions. The binding capacity of the test polymer is calculated as the linear slope of the calcium concentrations measured against the grams/L of test polymer that was added.

The dispersant polymer preferably bears a negative net charge when dissolved in an aqueous solution with a pH greater than 6.

The dispersant polymer can bear also sulfonated carboxylic esters or amides, in order to increase the negative charge at lower pH and improve their dispersing properties in hard water. The preferred dispersant polymers are sulfonated / carboxylated polymers, i.e., polymer comprising both sulfonated and carboxylated monomers.

Preferably, the dispersant polymers are sulfonated derivatives of polycarboxylic acids and may comprise two, three, four or more different monomer units. The preferred copolymers contain:

At least one structural unit derived from a carboxylic acid monomer having the general formula (III): 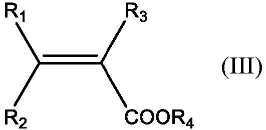 wherein R₁ to R₃ are independently selected from hydrogen, methyl, linear or branched saturated alkyl groups having from 2 to 12 carbon atoms, linear or branched mono or polyunsaturated alkenyl groups having from 2 to 12 carbon atoms, alkyl or alkenyl groups as aforementioned substituted with -NH2 or -OH, or -COOH, or COOR₄, where R₄ is selected from hydrogen, alkali metal, or a linear or branched, saturated or unsaturated alkyl or alkenyl group with 2 to 12 carbons;

Preferred carboxylic acid monomers include one or more of the following: acrylic acid, maleic acid, maleic anhydride, itaconic acid, citraconic acid, 2-phenylacrylic acid, cinnamic acid, crotonic acid, fumaric acid, methacrylic acid, 2-ethylacrylic acid, methylenemalonic acid, or sorbic acid. Acrylic and methacrylic acids being more preferred.

Optionally, one or more structural units derived from at least one nonionic monomer having the general formula (IV):

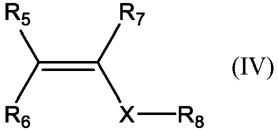

Wherein R₅ to R₇ are independently selected from hydrogen, methyl, phenyl or hydroxyalkyl groups containing 1 to 6 carbon atoms, and can be part of a cyclic structure, X is an optionally present spacer group which is selected from -CH₂-, -COO-, -CONH- or -CONR₈-, and R₈ is selected from linear or branched, saturated alkyl radicals having 1 to 22 carbon atoms or unsaturated, preferably aromatic, radicals having from 6 to 22 carbon atoms.

Preferred non-ionic monomers include one or more of the following: butene, isobutene, pentene, 2-methylpent-1-ene, 3-methylpent-1-ene, 2,4,4-trimethylpent-1-ene, 2,4,4-trimethylpent-2-ene, cyclopentene, methylcyclopentene, 2-methyl-3-methyl-cyclopentene, hexene, 2,3-dimethylhex-1-ene, 2,4-dimethylhex-1-ene, 2,5-dimethylhex-1-ene, 3,5-dimethylhex-1-ene, 4,4-dimethylhex-1-ene, cyclohexene, methylcyclohexene, cycloheptene, alpha olefins having 10 or more carbon atoms such as, dec-1-ene, dodec-1-ene, hexadec-1-ene, octadec-1-ene and docos-1-ene, preferred aromatic monomers are styrene, alpha methylstyrene, 3-methylstyrene, 4-dodecylstyrene, 2-ethyl-4-bezylstyrene, 4-cyclohexylstyrene, 4-propylstyrol, 1-vinylnaphtalene, 2-vinylnaphtalene; preferred carboxylic ester monomers are methyl (meth)acrylate, ethyl (meth)acrylate, propyl (meth)acrylate, t-butyl (meth)acrylate, pentyl (meth)acrylate, hexyl (meth)acrylate, 2-ethylhexyl (meth)acrylate, octyl (meth)acrylate, lauryl (meth)acrylate, stearyl (meth)acrylate and behenyl (meth)acrylate; preferred amides are N-methyl acrylamide, N-ethyl acrylamide, N-t-butyl acrylamide, N-2-ethylhexyl acrylamide, N-octyl acrylamide, N-lauryl acrylamide, N-stearyl acrylamide, N-behenyl acrylamide.
and at least one structural unit derived from at least one sulfonic acid monomer having the general formula (V) and (VI): 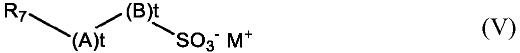 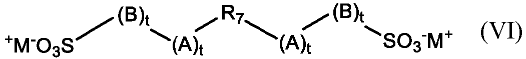
wherein R₇ is a group comprising at least one sp2 bond, A is O, N, P, S, an amido or ester linkage, B is a mono- or polycyclic aromatic group or an aliphatic group, each t is independently 0 or 1, and M+ is a cation. In one aspect, R₇ is a C2 to C6 alkene. In another aspect, R7 is ethene, butene or propene.

Preferred sulfonated monomers include one or more of the following: 1-acrylamido-1-propanesulfonic acid, 2-acrylamido-2-propanesulfonic acid, 2-acrylamido-2-methyl-1-propanesulfonic acid, 2-methacrylamido-2-methyl-1-propanesulfonic acid, 3- methacrylamido-2-hydroxy-propanesulfonic acid, allylsulfonic acid, methallylsulfonic acid, allyloxybenzenesulfonic acid, methallyloxybenzenesulfonic acid, 2-hydroxy-3- (2-propenyloxy) propanesulfonic acid, 2-methyl-2-propen-1-sulfonic acid, styrenesulfonic acid, vinylsulfonic acid, 3-sulfopropyl, 3-sulfo-propylmethacrylate, sulfomethacrylamide, sulfomethylmethacrylamide and mixtures of said acids or their water-soluble salts.

Preferably, the polymer comprises the following levels of monomers: from about 40 to about 90%, preferably from about 60 to about 90% by weight of the polymer of one or more carboxylic acid monomer; from about 5 to about 50%, preferably from about 10 to about 40% by weight of the polymer of one or more sulfonic acid monomer; and optionally from about 1% to about 30%, preferably from about 2 to about 20% by weight of the polymer of one or more non-ionic monomer. An especially preferred polymer comprises about 70% to about 80% by weight of the polymer of at least one carboxylic acid monomer and from about 20% to about 30% by weight of the polymer of at least one sulfonic acid monomer.

In the polymers, all or some of the carboxylic or sulfonic acid groups can be present in neutralized form, i.e. the acidic hydrogen atom of the carboxylic and/or sulfonic acid group in some or all acid groups can be replaced with metal ions, preferably alkali metal ions and in particular with sodium ions.

The carboxylic acid is preferably (meth)acrylic acid. The sulfonic acid monomer is preferably 2-acrylamido-2-propanesulfonic acid (AMPS).

Preferred commercial available polymers include: Alcosperse 240, Aquatreat AR 540 and Aquatreat MPS supplied by Alco Chemical; Acumer 3100, Acumer 2000, Acusol 587G and Acusol 588G supplied by Rohm & Haas; Goodrich K-798, K-775 and K-797 supplied by BF Goodrich; and ACP 1042 supplied by ISP technologies Inc. Particularly preferred polymers are Acusol 587G and Acusol 588G supplied by Rohm & Haas.

Suitable dispersant polymers include anionic carboxylic polymer of low molecular weight. They can be homopolymers or copolymers with a weight average molecular weight of less than or equal to about 200,000 g/mol, or less than or equal to about 75,000 g/mol, or less than or equal to about 50,000 g/mol, or from about 3,000 to about 50,000 g/mol, preferably from about 5,000 to about 45,000 g/mol. The dispersant polymer may be a low molecular weight homopolymer of polyacrylate, with an average molecular weight of from 1,000 to 20,000, particularly from 2,000 to 10,000, and particularly preferably from 3,000 to 5,000.

The dispersant polymer may be a copolymer of acrylic with methacrylic acid, acrylic and/or methacrylic with maleic acid, and acrylic and/or methacrylic with fumaric acid, with a molecular weight of less than 70,000. Their molecular weight ranges from 2,000 to 80,000 and more preferably from 20,000 to 50,000 and in particular 30,000 to 40,000 g/mol. and a ratio of (meth)acrylate to maleate or fumarate segments of from 30:1 to 1:2.

The dispersant polymer may be a copolymer of acrylamide and acrylate having a molecular weight of from 3,000 to 100,000, alternatively from 4,000 to 20,000, and an acrylamide content of less than 50%, alternatively less than 20%, by weight of the dispersant polymer can also be used. Alternatively, such dispersant polymer may have a molecular weight of from 4,000 to 20,000 and an acrylamide content of from 0% to 15%, by weight of the polymer.

Dispersant polymers suitable herein also include itaconic acid homopolymers and copolymers. Alternatively, the dispersant polymer can be selected from the group consisting of alkoxylated polyalkyleneimines, alkoxylated polycarboxylates, polyethylene glycols, styrene co-polymers, cellulose sulfate esters, carboxylated polysaccharides, amphiphilic graft copolymers and mixtures thereof.

### Bleach

The composition of the invention preferably comprises from about 10 to about 20%, more preferably from about 12 to about 18% of bleach, preferably percarbonate, by weight of the composition.

Inorganic and organic bleaches are suitable for use herein. Inorganic bleaches include perhydrate salts such as perborate, percarbonate, perphosphate, persulfate and persilicate salts. The inorganic perhydrate salts are normally the alkali metal salts. The inorganic perhydrate salt may be included as the crystalline solid without additional protection. Alternatively, the salt can be coated. Suitable coatings include sodium sulphate, sodium carbonate, sodium silicate and mixtures thereof. Said coatings can be applied as a mixture applied to the surface or sequentially in layers.

Alkali metal percarbonates, particularly sodium percarbonate is the preferred bleach for use herein. The percarbonate is most preferably incorporated into the products in a coated form which provides in-product stability.

Potassium peroxymonopersulfate is another inorganic perhydrate salt of utility herein.

Typical organic bleaches are organic peroxyacids, especially dodecanediperoxoic acid, tetradecanediperoxoic acid, and hexadecanediperoxoic acid. Mono- and diperazelaic acid, mono- and diperbrassylic acid are also suitable herein. Diacyl and Tetraacylperoxides, for instance dibenzoyl peroxide and dilauroyl peroxide, are other organic peroxides that can be used in the context of this invention.

Further typical organic bleaches include the peroxyacids, particular examples being the alkylperoxy acids and the arylperoxy acids. Preferred representatives are (a) peroxybenzoic acid and its ring-substituted derivatives, such as alkylperoxybenzoic acids, but also peroxy-α-naphthoic acid and magnesium monoperphthalate, (b) the aliphatic or substituted aliphatic peroxy acids, such as peroxylauric acid, peroxystearic acid, ε-phthalimidoperoxycaproic acid[phthaloiminoperoxyhexanoic acid (PAP)], o-carboxybenzamidoperoxycaproic acid, N-nonenylamidoperadipic acid and N-nonenylamidopersuccinates, and (c) aliphatic and araliphatic peroxydicarboxylic acids, such as 1,12-diperoxycarboxylic acid, 1,9-diperoxyazelaic acid, diperoxysebacic acid, diperoxybrassylic acid, the diperoxyphthalic acids, 2-decyldiperoxybutane-1,4-dioic acid, N,N-terephthaloyldi(6-aminopercaproic acid).

### Bleach Activators

Bleach activators are typically organic peracid precursors that enhance the bleaching action in the course of cleaning at temperatures of 60° C and below. Bleach activators suitable for use herein include compounds which, under perhydrolysis conditions, give aliphatic peroxoycarboxylic acids having preferably from 1 to 12 carbon atoms, in particular from 2 to 10 carbon atoms, and/or optionally substituted perbenzoic acid. Suitable substances bear O-acyl and/or N-acyl groups of the number of carbon atoms specified and/or optionally substituted benzoyl groups. Preference is given to polyacylated alkylenediamines, in particular tetraacetylethylenediamine (TAED), acylated triazine derivatives, in particular 1,5-diacetyl-2,4-dioxohexahydro-1,3,5-triazine (DADHT), acylated glycolurils, in particular tetraacetylglycoluril (TAGU), N-acylimides, in particular N-nonanoylsuccinimide (NOSI), acylated phenolsulfonates, in particular n-nonanoyl- or isononanoyloxybenzenesulfonate (n- or iso-NOBS), decanoyloxybenzoic acid (DOBA), carboxylic anhydrides, in particular phthalic anhydride, acylated polyhydric alcohols, in particular triacetin, ethylene glycol diacetate and 2,5-diacetoxy-2,5-dihydrofuran and also triethylacetyl citrate (TEAC). If present the composition of the invention comprises from 0.01 to 5, preferably from 0.2 to 2% by weight of the composition of bleach activator, preferably TAED.

### Bleach Catalyst

The composition herein preferably contains a bleach catalyst, preferably a metal containing bleach catalyst. More preferably the metal containing bleach catalyst is a transition metal containing bleach catalyst, especially a manganese or cobalt-containing bleach catalyst.

Bleach catalysts preferred for use herein include manganese triazacyclononane and related complexes; Co, Cu, Mn and Fe bispyridylamine and related complexes; and pentamine acetate cobalt(III) and related complexes. Especially preferred bleach catalyst for use herein are 1,4,7-trimethyl-1,4,7-triazacyclononane (Me-TACN) and 1,2, 4,7- tetramethyl-1,4,7-triazacyclononane (Me/Me-TACN).

Preferably the composition of the invention comprises from 0.005 to 0.5, more preferably from 0.005 to 0.075% of bleach catalyst by weight of the composition. Preferably the bleach catalyst is a manganese bleach catalyst.

### Inorganic builder

The composition of the invention preferably comprises an inorganic builder. Suitable inorganic builders are selected from the group consisting of carbonate, silicate and mixtures thereof. Especially preferred for use herein is sodium carbonate. Preferably the composition of the invention comprises from 5 to 60%, more preferably from 10 to 50% and especially from 15 to 45% of sodium carbonate by weight of the composition.

### Surfactant

Surfactants suitable for use herein include non-ionic surfactants, preferably the compositions are free of any other surfactants. Traditionally, non-ionic surfactants have been used in automatic dishwashing for surface modification purposes in particular for sheeting to avoid filming and spotting and to improve shine. It has been found that non-ionic surfactants can also contribute to prevent redeposition of soils.

Preferably the composition of the invention comprises a non-ionic surfactant or a non-ionic surfactant system, more preferably the non-ionic surfactant or a non-ionic surfactant system has a phase inversion temperature, as measured at a concentration of 1% in distilled water, between 40 and 70°C, preferably between 45 and 65°C. By a "non-ionic surfactant system" is meant herein a mixture of two or more non-ionic surfactants. Preferred for use herein are non-ionic surfactant systems. They seem to have improved cleaning and finishing properties and better stability in product than single non-ionic surfactants.

Phase inversion temperature is the temperature below which a surfactant, or a mixture thereof, partitions preferentially into the water phase as oil-swollen micelles and above which it partitions preferentially into the oil phase as water swollen inverted micelles. Phase inversion temperature can be determined visually by identifying at which temperature cloudiness occurs.

The phase inversion temperature of a non-ionic surfactant or system can be determined as follows: a solution containing 1% of the corresponding surfactant or mixture by weight of the solution in distilled water is prepared. The solution is stirred gently before phase inversion temperature analysis to ensure that the process occurs in chemical equilibrium. The phase inversion temperature is taken in a thermostable bath by immersing the solutions in 75 mm sealed glass test tube. To ensure the absence of leakage, the test tube is weighed before and after phase inversion temperature measurement. The temperature is gradually increased at a rate of less than 1°C per minute, until the temperature reaches a few degrees below the pre-estimated phase inversion temperature. Phase inversion temperature is determined visually at the first sign of turbidity.

Suitable nonionic surfactants include: i) ethoxylated non-ionic surfactants prepared by the reaction of a monohydroxy alkanol or alkyphenol with 6 to 20 carbon atoms with preferably at least 12 moles particularly preferred at least 16 moles, and still more preferred at least 20 moles of ethylene oxide per mole of alcohol or alkylphenol; ii) alcohol alkoxylated surfactants having a from 6 to 20 carbon atoms and at least one ethoxy and propoxy group. Preferred for use herein are mixtures of surfactants i) and ii).

Another suitable non-ionic surfactants are epoxy-capped poly(oxyalkylated) alcohols represented by the formula:

R1O[CH2CH(CH3)O]x[CH2CH2O]y[CH2CH(OH)R2] (I)

wherein R1 is a linear or branched, aliphatic hydrocarbon radical having from 4 to 18 carbon atoms; R2 is a linear or branched aliphatic hydrocarbon radical having from 2 to 26 carbon atoms; x is an integer having an average value of from 0.5 to 1.5, more preferably about 1; and y is an integer having a value of at least 15, more preferably at least 20.

Preferably, the surfactant of formula I, at least about 10 carbon atoms in the terminal epoxide unit [CH2CH(OH)R2]. Suitable surfactants of formula I, according to the present invention, are Olin Corporation's POLY-TERGENT^{®} SLF-18B nonionic surfactants, as described, for example, in WO 94/22800, published October 13, 1994 by Olin Corporation.

### Enzymes

### Other proteases

The composition of the invention can comprise a protease in addition to the protease of the invention. A mixture of two or more proteases can contribute to an enhanced cleaning across a broader temperature, cycle duration, and/or substrate range, and provide superior shine benefits, especially when used in conjunction with an anti-redeposition agent and/or a sulfonated polymer.

Suitable proteases for use in combination with the variant proteases of the invention include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:
(a) subtilisins (EC 3.4.21.62), especially those derived from Bacillus, such as Bacillus sp., B. lentus, B. alkalophilus, B. subtilis, B. amyloliquefaciens, B. pumilus , B. gibsonii, and B. akibaii described in WO2004067737, WO2015091989, WO2015091990, WO2015024739, WO2015143360, US 6,312,936 B1, US 5,679,630, US 4,760,025, DE102006022216A1, DE102006022224A1, WO2015089447, WO2015089441, WO2016066756, WO2016066757, WO2016069557, WO2016069563, WO2016069569.
(b) trypsin-type or chymotrypsin-type proteases, such as trypsin (e.g., of porcine or bovine origin), including the Fusarium protease described in WO 89/06270 and the chymotrypsin proteases derived from Cellumonas described in WO 05/052161 and WO 05/052146.
(c) metalloproteases, especially those derived from Bacillus amyloliquefaciens decribed in WO07/044993A2; from Bacillus, Brevibacillus, Thermoactinomyces, Geobacillus, Paenibacillus, Lysinibacillus or Streptomyces spp. Described in WO2014194032, WO2014194054 and WO2014194117; from Kribella alluminosa described in WO2015193488; and from Streptomyces and Lysobacter described in WO2016075078.
(d) protease having at least 90% identity to the subtilase from Bacillus sp. TY145, NCIMB 40339, described in WO92/17577 (Novozymes A/S), including the variants of this Bacillus sp TY145 subtilase described in WO2015024739, and WO2016066757.

Especially preferred additional proteases for the detergent of the invention are polypeptides demonstrating at least 90%, preferably at least 95%, more preferably at least 98%, even more preferably at least 99% and especially 100% identity with the wild-type enzyme from Bacillus lentus, comprising mutations in one or more, preferably two or more and more preferably three or more of the following positions, using the BPN' numbering system and amino acid abbreviations as illustrated in WO00/37627:V68A, N76D, N87S, S99D, S99SD, S99A, S101G, S101M, S103A, V104N/I, G118V, G118R, S128L, P129Q, S130A, Y167A, R170S, A194P, V205I, Q206L/D/E, Y209W and/or M222S.

Most preferably the additional protease is selected from the group of proteases comprising the below mutations (BPN' numbering system) versus either the PB92 wild-type (SEQ ID NO:2 in WO 08/010925) or the subtilisin 309 wild-type (sequence as per PB92 backbone, except comprising a natural variation of N87S).
(i) G118V + S128L + P129Q + S130A
(ii) S101M + G118V + S128L + P129Q + S130A
(iii) N76D + N87R + G118R + S128L + P129Q + S130A + S188D + N248R
(iv) N76D + N87R + G118R + S128L + P129Q + S130A + S188D + V244R
(v) N76D + N87R + G118R + S128L + P129Q + S130A
(vi) V68A + N87S + S101G + V104N
(vii) S99AD

Suitable commercially available additional protease enzymes include those sold under the trade names Alcalase^{®}, Savinase^{®}, Primase^{®}, Durazym^{®}, Polarzyme^{®}, Kannase^{®}, Liquanase^{®}, Liquanase Ultra^{®}, Savinase Ultra^{®}, Ovozyme^{®}, Neutrase^{®}, Everlase^{®}, Coronase^{®}, Blaze^{®}, Blaze Ultra^{®} and Esperase^{®} by Novozymes A/S (Denmark); those sold under the tradename Maxatase^{®}, Maxacal^{®}, Maxapem^{®}, Properase^{®}, Purafect^{®}, Purafect Prime^{®}, Purafect Ox^{®}, FN3^{®}, FN4^{®}, Excellase^{®}, Ultimase^{®} and Purafect OXP^{®} by Dupont; those sold under the tradename Opticlean^{®} and Optimase^{®} by Solvay Enzymes; and those available from Henkel/Kemira, namely BLAP (sequence shown in Figure29 of US 5,352,604 with the following mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D); and KAP (Bacillus alkalophilus subtilisin with mutations A230V + S256G + S259N) from Kao.

Especially preferred for use herein in combination with the variant protease of the invention are commercial proteases selected from the group consisting of Properase^{®}, Blaze^{®}, Ultimase^{®}, Everlase^{®}, Savinase^{®}, Excellase^{®}, Blaze Ultra^{®}, BLAP and BLAP variants.

Preferred levels of protease in the product of the invention include from about 0.05 to about 10, more preferably from about 0.5 to about 7 and especially from about 1 to about 6 mg of active protease/g of composition.

### Amylases

Preferably the composition of the invention may comprise an amylase. Suitable alpha-amylases include those of bacterial or fungal origin. Chemically or genetically modified mutants (variants) are included. A preferred alkaline alpha-amylase is derived from a strain of Bacillus, such as Bacillus licheniformis, Bacillus amyloliquefaciens, Bacillus stearothermophilus, Bacillus subtilis, or other Bacillus sp., such as Bacillus sp. NCBI 12289, NCBI 12512, NCBI 12513, DSM 9375 (USP 7,153,818) DSM 12368, DSMZ no. 12649, KSM AP1378 (WO 97/00324), KSM K36 or KSM K38 (EP 1,022,334). Preferred amylases include:
(a) variants described in WO 96/23873, WO00/60060, WO06/002643 and WO2017/192657, especially the variants with one or more substitutions in the following positions versus the AA560 enzyme listed as SEQ ID NO. 12 in WO06/002643:
   26, 30, 33, 82, 37, 106, 118, 128, 133, 149, 150, 160, 178, 182, 186, 193, 202, 214, 231, 246, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, preferably that also contain the deletions of D183* and G184*.
(b) variants exhibiting at least 90% identity with SEQ ID No. 4 in WO06/002643, the wild-type enzyme from Bacillus SP722, especially variants with deletions in the 183 and 184 positions and variants described in WO 00/60060, WO2011/100410 and WO2013/003659.
(c) variants exhibiting at least 95% identity with the wild-type enzyme from Bacillus sp.707 (SEQ ID NO:7 in US 6,093, 562), especially those comprising one or more of the following mutations M202, M208, S255, R172, and/or M261. Preferably said amylase comprises one or more of M202L, M202V, M202S, M202T, M202I, M202Q, M202W, S255N and/or R172Q. Particularly preferred are those comprising the M202L or M202T mutations.
(d) variants described in WO 09/149130, preferably those exhibiting at least 90% identity with SEQ ID NO: 1 or SEQ ID NO:2 in WO 09/149130, the wild-type enzyme from Geobacillus Stearophermophilus or a truncated version thereof.
(e) variants exhibiting at least 89% identity with SEQ ID NO:1 in WO2016091688, especially those comprising deletions at positions H183+G184 and additionally one or more mutations at positions 405, 421, 422 and/or 428.
(f) variants exhibiting at least 60% amino acid sequence identity with the "PcuAmyl α-amylase" from Paenibacillus curdlanolyticus YK9 (SEQ ID NO:3 in WO2014099523).
(g) variants exhibiting at least 60% amino acid sequence identity with the "CspAmy2 amylase" from Cytophaga sp. (SEQ ID NO:1 in WO2014164777).
(h) variants exhibiting at least 85% identity with AmyE from Bacillus subtilis (SEQ ID NO:1 in WO2009149271).
(i) variants exhibiting at least 90% identity with the wild-type amylase from Bacillus sp. KSM-K38 with accession number AB051102.
(j) variants exhibiting at least 80% identity with the mature amino acid sequence of AAI10 from Bacillus sp (SEQ ID NO:7 in WO2016180748)
(k) variants exhibiting at least 80% identity with the mature amino acid sequence of Alicyclobacillus sp. amylase (SEQ ID NO:8 in WO2016180748)

Preferably the amylase is an engineered enzyme, wherein one or more of the amino acids prone to bleach oxidation have been substituted by an amino acid less prone to oxidation. In particular it is preferred that methionine residues are substituted with any other amino acid. In particular it is preferred that the methionine most prone to oxidation is substituted. Preferably the methionine in a position equivalent to 202 in the AA560 enzyme listed as SEQ ID NO. 12 in WO06/002643 is substituted. Preferably, the methionine at this position is substituted with threonine or leucine, preferably leucine.

Suitable commercially available alpha-amylases include DURAMYL^{®}, LIQUEZYME^{®}, TERMAMYL^{®}, TERMAMYL ULTRA^{®}, NATALASE^{®}, SUPRAMYL^{®}, STAINZYME^{®}, STAINZYME PLUS^{®}, FUNGAMYL^{®}, ATLANTIC^{®}, INTENSA^{®} and BAN^{®} (Novozymes A/S, Bagsvaerd, Denmark), KEMZYM^{®} AT 9000 Biozym Biotech Trading GmbH Wehlistrasse 27b A-1200 Wien Austria, RAPIDASE^{®} , PURASTAR9, ENZYSIZE^{®}, OPTISIZE HT PLUS^{®}, POWERASE^{®}, PREFERENZ S^{®} series (including PREFERENZ S1000^{®} and PREFERENZ S2000^{®} and PURASTAR OXAM^{®} (DuPont., Palo Alto, California) and KAM^{®} (Kao, 14-10 Nihonbashi Kayabacho, 1-chome, Chuo-ku Tokyo 103-8210, Japan). In one aspect, suitable amylases include ATLANTIC^{®}, STAINZYME^{®}, POWERASE^{®}, INTENSA^{®} and STAINZYME PLUS^{®} and mixtures thereof.

Preferably, the product of the invention comprises at least 0.01 mg, preferably from about 0.05 to about 10, more preferably from about 0.1 to about 6, especially from about 0.2 to about 5 mg of active amylase/ g of composition.

Preferably, the protease and/or amylase of the composition of the invention are in the form of granulates, the granulates comprise more than 29% of sodium sulfate by weight of the granulate and/or the sodium sulfate and the active enzyme (protease and/or amylase) are in a weight ratio of between 3:1 and 100:1 or preferably between 4:1 and 30:1 or more preferably between 5:1 and 20:1.

### Crystal growth inhibitor

Crystal growth inhibitors are materials that can bind to calcium carbonate crystals and prevent further growth of species such as aragonite and calcite.

Examples of effective crystal growth inhibitors include phosphonates, polyphosphonates, inulin derivatives, polyitaconic acid homopolymers and cyclic polycarboxylates.

Suitable crystal growth inhibitors may be selected from the group comprising HEDP (1-hydroxyethylidene 1,1-diphosphonic acid), carboxymethylinulin (CMI), tricarballylic acid and cyclic carboxylates. For the purposes of this invention the term carboxylate covers both the anionic form and the protonated carboxylic acid form.

Cyclic carboxylates contain at least two, preferably three or preferably at least four carboxylate groups and the cyclic structure is based on either a mono- or bi-cyclic alkane or a heterocycle. Suitable cyclic structures include cyclopropane, cyclobutane, cyclohexane or cyclopentane or cycloheptane, bicyclo-heptane or bicyclo-octane and/or tetrhaydrofuran. One preferred crystal growth inhibitor is cyclopentane tetracarboxylate.

Cyclic carboxylates having at least 75%, preferably 100% of the carboxylate groups on the same side, or in the "cis" position of the 3D-structure of the cycle are preferred for use herein.

It is preferred that the two carboxylate groups, which are on the same side of the cycle are in directly neighbouring or "ortho" positions.

Preferred crystal growth inhibitors include HEDP, tricarballylic acid, tetrahydrofurantetracarboxylic acid (THFTCA) and cyclopentanetetracarboxylic acid (CPTCA). The THFTCA is preferably in the 2c,3t,4t,5c-configuration, and the CPTCA in the cis,cis,cis,cis-configuration. Especially preferred crystal growth inhibitor for use herein is HEDP.

Also preferred for use herein are partially decarboxylated polyitaconic acid homopolymers, preferably having a level of decarboxylation is in the range of 50 mole % to 90 mole %. Especially preferred polymer for use herein is Itaconix TSI^{®} provided by Itaconix.

The crystal growth inhibitors are present preferably in a quantity from about 0.01 to about 10 %, particularly from about 0.02 to about 5 % and in particular, from 0.05 to 3 % by weight of the composition.

### Metal Care Agents

Metal care agents may prevent or reduce the tarnishing, corrosion or oxidation of metals, including aluminium, stainless steel and non-ferrous metals, such as silver and copper. Preferably the composition of the invention comprises from 0.1 to 5%, more preferably from 0.2 to 4% and especially from 0.3 to 3% by weight of the product of a metal care agent, preferably the metal care agent is benzo triazole (BTA).

### Glass Care Agents

Glass care agents protect the appearance of glass items during the dishwashing process. Preferably the composition of the invention comprises from 0.1 to 5%, more preferably from 0.2 to 4% and specially from 0.3 to 3% by weight of the composition of a metal care agent, preferably the glass care agent is a zinc containing material, specially hydrozincite. Other suitable glass care agents are polyethyleneimine (PEI). A particularly preferred PEI is Lupasol^{®} FG, supplied by BASF.

The automatic dishwashing composition of the invention preferably has a pH as measured in 1% weight/volume aqueous solution in distilled water at 20°C of from about 9 to about 12, more preferably from about 10 to less than about 11.5 and especially from about 10.5 to about 11.5. The automatic dishwashing composition of the invention preferably has a reserve alkalinity of from about 10 to about 20, more preferably from about 12 to about 18 at a pH of 9.5 as measured in NaOH with 100 grams of product at 20°C.

A preferred automatic dishwashing composition of the invention comprises:
i) from 10 to 20% by weight of the composition of bleach, preferably sodium percarbonate;
ii) preferably a bleach activator, more preferably TAED;
iii) amylases;
iv) optionally but preferably from 5 to 50% by weight of the composition of an inorganic builder, preferably sodium carbonate;
v) optionally but preferably from 2 to 10% by weight of the composition of a non-ionic surfactant;
vi) optionally but preferably a bleach catalyst, more preferably a manganese bleach catalyst; and
vii) other optional ingredients include: a crystal growth inhibitor, preferably HEDP, and glass care agents.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

### EXAMPLES

Egg yolk removal by automatic dishwashing compositions comprising variant proteases were compared with the same compositions comprising the parent protease.

The compositions displayed in Table 1 were used. 3 g of each composition were dissolved in a litre of deionized water to produce a cleaning solution having a pH of 11. The corresponding protease was added to the cleaning solution at a level between 0.25-0.75ppm.

**Table 1: Automatic Dishwashing Compositions**

| **Ingredients (active weight %)** | **ADW Formula A** | **ADW Formula B** | **ADW Formula C** |
|---|---|---|---|
| **Solid ingredients** | | | |
| Sodium carbonate | 41.7 | 41.7 | 41.7 |
| Sodium sulphate | 0.00 | 1.68 | 2.03 |
| MGDA | 21.0 | 0.00 | 10.1 |
| Sodium citrate | 0.00 | 19.2 | 10.1 |
| TAED | 1.68 | 1.68 | 1.68 |
| Sodium percarbonate | 12.6 | 12.6 | 12.6 |
| Sulfonated polymer | 2.5 | 2.5 | 2.5 |
| Bleach catalyst | 1.2 | 1.2 | 1.2 |
| Amylase | 0.11 | 0.11 | 0.11 |

| **Liquid ingredients** | | | |
|---|---|---|---|
| Lutensol TO7 | 19.3 | 19.3 | 19.3 |

**Table 1: Automatic Dish Washing (ADW) Compositions**

| | |
|---|---|
| Amylase | Stainzyme^{®} Plus supplied by Novozymes |
| TAED | Tetraacetylethylenediamine |
| MGDA | Three-sodium methyl glycine diacetate supplied by BASF |
| Bleach catalyst | MnTACN (Manganese 1,4,7-Triazacyclononane) |
| Sulfonated polymer | Acusol 588 supplied by Dow Chemicals |
| Lutensol TO7 | Nonionic surfactant supplied by BASF |

### Cleaning Performance Method: Egg Yolk cleaning performance using PAS-38

The cleaning performance of the proteases variants listed in Table 2 was tested relative to the parent using Automatic Dishwashing Compositions A, B and C (see Table 1), as measured by the stain removal on egg yolk microswatches (PAS-38, Center for Testmaterials BV, Vlaardingen, Netherlands). The egg swatch stains were pre-sized to fit the microtiter plate (MTPs); standard 96 well plate. The stain removal of the PAS-38 egg swatches was measured post wash versus a reference.

The MTPs were filled prior to protease addition with 3 g/l of detergent and the detergent and deionized water.

After incubating the PAS-38 swatches for 30°C min at 50°C, absorbance was read at 405 nm with a SpectraMax plate reader. Absorbance results were obtained by subtracting the value for a blank control (containing no protease) from each sample value (hereinafter "blank subtracted absorbance"). For each condition and variant, a performance index (PI) was calculated by dividing the blank subtracted absorbance by that of the parent protease at the same concentration. The value for the parent protease was determined from a standard curve of the parent protease which was included in the test and which was fitted to a Langmuir fit.

### Protease Enzyme Activity Method 2: AAPF Assay

The protease activity of parent and subtilisin variants thereof was tested by measuring hydrolysis of N-suc-AAPF-pNA. The reagent solutions used for the AAPF hydrolysis assay were: 100 mM Tris/HCl pH 8.6, containing 0.005% TWEEN^{®}-80 (Tris dilution buffer); 100 mM Tris buffer pH 8.6, containing 10 mM CaCl₂ and 0.005% TWEEN^{®}-80 (Tris/Ca buffer); and 160 mM suc-AAPF-pNA in DMSO (suc-AAPF-pNA stock solution) (Sigma: S-7388). A substrate working solution was prepared by adding 1 mL suc-AAPF-pNA stock solution to 100 mL Tris/Ca buffer and mixed well. An enzyme sample was added to a MTP (Greiner 781101) containing 1 mg/suc-AAPF-pNA working solution and assayed for activity at 405 nm over 3 min with a SpectraMax plate reader in kinetic mode at room temperature (RT). The absorbance of a blank containing no protease was subtracted from each sample reading. The protease activity was expressed as mOD-min⁻¹.

### Protease Enzyme Stability - Stability Assay

The stability of the variants described herein was measured by diluting the variants in stress buffer and measuring the proteolytic activity of the variants before and after a heat incubation step of 5 minutes at 56°C using the AAPF assay described above. Stability was measured in Tris-EDTA (50mM Tris pH 9; 5 mM EDTA; 0.005% Tween 80) buffered condition. % Residual activities were calculated by taking a ratio of the stressed to unstressed activity and multiplying by 100.

**Protease Data Table 2**

| | | **Stability** | **PAS-38 stain Cleaning** | | |
|---|---|---|---|---|---|
| **Variants** | **Substitutions with respect to SEQ ID No:1** | **TRIS-EDTA** | **ADW formula A** | **ADW formula B** | **ADW formula C** |
| SEQ ID No:1 | none | 1 | 1.0 | 1.0 | 1.0 |
| WALBSP-07063 (comparative) | T056Y-S099R-S126A-D127E-F128G | 9 | 1.7 | 2.2 | 2.6 |
| WALBSP-07078 (comparative) | S039E-I080V-S099R-S126A-D127E-F128G-M211L | 21 | 2.2 | 3.5 | ND |
| WALBSP-07137 (comparative) | S039E-P054T-S099R-S 126A-D127E-F128G-M211L | 28 | 2.3 | 2.9 | ND |
| WALBSP-07117 (comparative) | S039E-I043V-S099R-S 126A-D127E-F128G-M211L | 28 | 2.3 | 3.0 | 3.7 |
| WALBSP-07119 (comparative) | S039E-N042R-S099R-S126A-D127E-F128G | 28 | 2.3 | 3.1 | 3.9 |
| WALBSP-07052 (comparative) | S039E-I080V-S099R-S126A-D127E-F128G | 29 | 2.1 | 2.5 | 2.8 |
| WALBSP-07097 (comparative) | S039E-S099R-S126A-D127E-F128G-M211L | 29 | 2.4 | 2.6 | ND |
| WALBSP-07088 (comparative) | S039E-N085S-S099R-S126A-D127E-F128G-M211L | 30 | 2.3 | 3.3 | 3.6 |
| WALBSP-07147 (comparative) | S039E-T056Y-S099R-S 126A-D127E-F128G-M211L | 31 | 2.0 | 3.5 | 3.8 |
| WALBSP-07127 (comparative) | S039E-A047V-S099R-S126A-D127E-F128G-M211L | 31 | 2.2 | 2.6 | 3.9 |
| WALBSP-07149 (comparative) | S039E-S099R-S126A-D127E-F128G-Y203W | 32 | 2.0 | 2.6 | ND |
| WALBSP-07107 (comparative) | A037T-S039E-S099R-S 126A-D127E-F128G-M211L | 32 | 2.3 | 3.3 | ND |
| WALBSP-07129 (comparative) | S039E-S099R-S126A-D127E-F128G-V199I | 34 | 2.3 | 3.1 | 4.3 |
| WALBSP-07058 (comparative) | A037T-S039E-I043V-A047V-P054T-T056Y-I080V-N085S-E087D-S099R-T114Q-S126A-D127E-F128G | 35 | 2.0 | 2.2 | 2.8 |
| WALBSP-07050 (comparative) | S039E-I043V-A047V-P054T-T056Y-I080V-N085S-E087D-S099R-S126A-D127E-F128G-N242D | 35 | 2.0 | 2.4 | 2.9 |
| WALBSP-07040 (comparative) | A037T-S039E-I043V-A047V-T056Y-I080V-N085S-E087D-S099R-T114Q-S126A-D127E-F128G | 36 | 2.0 | 2.7 | 3.1 |
| WALBSP-07014 (comparative) | A037T-S039E-I043V-A047V-P054T-T056Y-I080V-N085S-E087D-S099R-S126A-D127E-F128G | 37 | 3.6 | 3.4 | 3.9 |
| WALBSP-07012 (comparative) | S039E-I043V-S099R-S 126A-D127E-F128G | 38 | 2.2 | 2.1 | 2.7 |
| WALBSP-07080 (comparative) | S039E-S099R-S126A-D127E-F128G-N253D | 38 | 1.7 | 1.9 | ND |
| WALBSP-07109 (comparative) | T009E-S039E-S099R-S126A-D127E-F128G | 38 | 2.1 | 3.0 | 3.4 |
| WALBSP-07090 (comparative) | S039E-S099R-S126A-D127E-F128G-S255W | 38 | 2.4 | 2.9 | 3.8 |
| WALBSP-07041 (comparative) | A037T-S039E-I043V-A047V-P054T-T056Y-I080V-N085S-S099R-S126A-D127E-F128G-N242D | 38 | 2.0 | 2.5 | 2.9 |
| WALBSP-07001 (comparative) | A037T-S039E-I043V-P054T-T056Y-I080V-N085S-E087D-S099R-S126A-D127E-F128G-N242D | 38 | 1.9 | 2.4 | 3.0 |
| WALBSP-07024 (comparative) | S039E-I043V-A047V-T056Y-I080V-N085S-E087D-S099R-S126A-D127E-F128G-N242D | 39 | 2.1 | 2.3 | 2.5 |
| WALBSP-07068 (comparative) | S039E-I043V-A047V-T056Y-I080V-N085S-E087D-S099R-T114Q-S126A-D127E-F128G-N242D | 39 | 1.8 | 2.3 | 2.8 |
| WALBSP-07042 (comparative) | S039E-T056Y-S099R-S126A-D127E-F128G | 39 | 2.1 | 2.3 | 2.6 |
| WALBSP-07065 (comparative) | A037T-S039E-I043V-A047V-T056Y-I080V-N085S-E087D-S099R-S 126A-D127E-F128G | 39 | 2.1 | 1.9 | 2.7 |
| WALBSP-07027 (comparative) | A037T-S039E-I043V-P054T-T056Y-I080V-N085S-E087D-S099R-T114Q-S126A-D127E-F128G-N242D | 39 | 1.9 | 2.5 | 3.0 |
| WALBSP-07019 (comparative) | A037T-S039E-I043V-T056Y-I080V-N085S-E087D-S099R-T114Q-S126A-D127E-F128G-N242D | 39 | 2.1 | 2.4 | 2.9 |
| WALBSP-07029 (comparative) | A037T-S039E-I043V-A047V-I080V-N085S-E087D-S099R-T114Q-S126A-D127E-F128G-N242D | 39 | 1.9 | 2.5 | 3.0 |
| WALBSP-07011 (comparative) | A037T-S039E-I043V-A047V-P054T-I080V-N085S-E087D-S099R-S126A-D127E-F128G-N242D | 39 | 2.0 | 2.2 | 2.8 |
| WALBSP-07055 (comparative) | A037T-S039E-I043V-A047V-T056Y-I080V-N085S-E087D-S099R-S126A-D127E-N242D | 39 | 2.0 | 2.1 | 2.6 |
| WALBSP-07054 (comparative) | A037T-S039E-I043V-T056Y-I080V-N085S-E087D-S099R-S126A-D127E-F128G-N242D | 39 | 2.0 | 2.1 | 2.7 |
| WALBSP-07030 (comparative) | A037T-S039E-I043V-A047V-T056Y-I080V-N085S-E087D-S099R-T114Q-S126A-D127EN242D | 39 | 2.0 | 2.4 | 3.0 |
| WALBSP-07062 (comparative) | S039E-N085S-S099R-S126A-D127E-F128G | 40 | 2.0 | 2.5 | 2.7 |
| WALBSP-07047 (comparative) | A037T-S039E-I043V-A047V-P054T-I080V-N085S-E087D-S099R-T114Q-S126A-D127E-F128G-N242D | 40 | 1.9 | 2.5 | 2.8 |
| WALBSP-06998 (comparative) | A037T-S039E-I043V-A047V-P054T-T056Y-I080V-N085S-S099R-T114Q-S126A-D127E-F128G-N242D | 40 | 1.9 | 2.4 | 2.7 |
| WALBSP-07048 (comparative) | A037T-S039E-I043V-A047V-P054T-T056Y-I080V-N085S-E087D-S099R-T114Q-S126A-D127E-N242D | 40 | 2.0 | 2.3 | 3.0 |
| WALBSP-07051 (comparative) | A037T-S039E-I043V-A047V-P054T-T056Y-I080V-N085S-E087D-S 126A-D127E-F128G-N242D | 40 | 0.7 | 0.7 | 0.8 |
| WALBSP-07045 (comparative) | A037T-S039E-I043V-A047V-T056Y-I080V-N085S-E087D-S099R-S126A-F128G-N242D | 40 | 2.1 | 2.1 | 2.6 |
| WALBSP-07049 (comparative) | A037T-S039E-I043V-A047V-T056Y-I080V-E087D-S099R-T114Q-S126A-D127E-F128G-N242D | 41 | 2.0 | 2.6 | 2.9 |
| WALBSP-07003 (comparative) | S039E-S099R-T114Q-S126A-D127E-F128G | 41 | 2.1 | 2.3 | 2.8 |
| WALBSP-07004 (comparative) | A037T-S039E-I043V-A047V-P054T-T056Y-I080V-N085S-E087D-S099R-S126A-D127EN242D | 41 | 1.9 | 2.2 | 2.6 |
| WALBSP-07000 (comparative) | A037T-S039E-I043V-A047V-T056Y-I080V-N085S-E087D-S099R-S126A-D127E-F128G-N242D | 41 | 1.9 | 2.2 | 2.7 |
| WALBSP-07028 (comparative) | A037T-S039E-I043V-A047V-P054T-T056Y-I080V-N085S-E087D-S099R-T114Q-D127E-F128G-N242D | 41 | 2.1 | 2.2 | 2.7 |
| WALBSP-07059 (comparative) | A037T-S039E-I043V-A047V-T056Y-I080V-N085S-S099R-T114Q-S126A-D127E-F128G-N242D | 41 | 2.0 | 2.3 | 3.0 |
| WALBSP-07022 (comparative) | S039E-A047V-S099R-S 126A-D127E-F128G | 41 | 2.1 | 2.5 | 2.5 |
| WALBSP-07015 (comparative) | A037T-S039E-I043V-A047V-T056Y-I080V-N085S-S099R-S126A-D127E-F128G-N242D | 41 | 2.0 | 2.0 | 2.7 |
| WALBSP-07033 (comparative) | A037T-S039E-I043V-A047V-T056Y-S099R-T114Q-S126A-D127E-F128G | 41 | 2.1 | 2.4 | 2.6 |
| WALBSP-07031 (comparative) | A037T-S039E-I043V-A047V-P054T-T056Y-I080V-E087D-S099R-S126A-D127E-F128G-N242D | 41 | 2.0 | 2.3 | 2.8 |
| WALBSP-07032 (comparative) | S039E-P054T-S099R-S126A-D127E-F128G | 41 | 3.6 | 4.1 | 4.6 |
| WALBSP-04815 (inventive) | A37T-S39E-I43V-A47V-P54T-T56Y-I80V-N85S-E87D-S99R-T114Q-S126A-D127E-F128G-N242D | 36 | 2.0 | 2.3 | 2.6 |
| WALBSP-07064 (comparative) | A037T-S039E-I043V-A047V-I080V-N085S-E087D-S099R-S126A-D127E-F128G-N242D | 42 | 2.0 | 1.9 | 2.4 |
| WALBSP-07005 (comparative) | A037T-S039E-I043V-A047V-T056Y-I080V-E087D-S099R-S126A-D127E-F128G-N242D | 42 | 1.9 | 2.0 | 2.5 |
| WALBSP-07010 (comparative) | A037T-S039E-I043V-A047V-T056Y-I080V-N085S-E087D-S099R-T114Q-D127E-F128G-N242D | 42 | 2.1 | 2.6 | 2.9 |
| WALBSP-07061 (comparative) | A037T-S039E-I043V-A047V-P054T-T056Y-I080V-N085S-E087D-S099R-D127E-F128G-N242D | 42 | 2.0 | 2.1 | 2.9 |
| WALBSP-07072 (comparative) | S039E-E087D-S099R-S126A-D127E-F128G | 42 | 2.0 | 1.9 | 2.8 |
| WALBSP-07035 (comparative) | A037T-S039E-I043V-A047V-T056Y-I080V-N085S-E087D-S099R-D127E-F128G-N242D | 42 | 2.1 | 2.1 | 2.7 |
| WALBSP-07038 (comparative) | A037T-S039E-I043V-A047V-P054T-T056Y-I080V-N085S-E087D-S099R-T114Q-S126A-F128G-N242D | 43 | 1.9 | 2.2 | 2.8 |
| WALBSP-07002 (comparative) | A037T-S039E-S099R-S126A-D127E-F128G | 43 | 3.8 | 4.8 | 5.5 |
| WALBSP-07067 (comparative) | A037T-S039E-I043V-A047V-P054T-T056Y-I080V-E087D-S099R-T114Q-S126A-D127E-F128G-N242D | 43 | 1.8 | 2.5 | 2.9 |
| WALBSP-07018 (comparative) | A037T-S039E-I043V-A047V-P054T-T056Y-I080V-N085S-E087D-S099R-S126A-D127E-F128G-N242D | 43 | 1.9 | 2.2 | 2.8 |
| WALBSP-07020 (comparative) | A037T-S039E-I043V-A047V-T056Y-I080V-N085S-E087D-S099R-T114Q-S126A-F128G-N242D | 43 | 2.0 | 2.7 | 3.1 |
| WALBSP-07043 (comparative) | A037T-S039E-I043V-A047V-T056Y-S099R-S126A-D127E-F128G | 44 | 2.0 | 2.3 | 2.7 |
| WALBSP-07023 (comparative) | A037T-S039E-I043V-A047V-P054T-T056Y-S099R-T114Q-S126A-D127E-F128G | 44 | 2.1 | 2.4 | 2.7 |
| WALBSP-07070 (comparative) | A037T-S039E-A047V-P054T-T056Y-I080V-N085S-E087D-S099R-S126A-D127E-F128G-N242D | 44 | 2.0 | 2.1 | 2.9 |
| WALBSP-07071 (comparative) | A037T-S039E-I043V-A047V-P054T-T056Y-I080V-N085S-E087D-S099R-S 126A-F 128G-N242D | 44 | 1.9 | 2.2 | 3.0 |
| WALBSP-07008 (comparative) | A037T-S039E-I043V-A047V-P054T-T056Y-I080V-N085S-E087D-T114Q-S126A-D127E-F128G-N242D | 44 | 0.7 | 0.7 | 0.8 |
| WALBSP-07025 (comparative) | A037T-S039E-I043V-A047V-T056Y-I080V-N085S-E087D-S126A-D127E-F128G-N242D | 45 | 0.8 | 0.5 | 0.7 |
| WALBSP-07009 (comparative) | A037T-S039E-A047V-T056Y-I080V-N085S-E087D-S099R-T114Q-S126A-D127E-F128G-N242D | 45 | 2.1 | 2.6 | 3.2 |
| WALBSP-07075 (comparative) | S039E-S099R-S126A-D127E-F128G | 45 | 1.9 | 2.3 | 2.8 |
| WALBSP-07017 (comparative) | A037T-S039E-A047V-P054T-T056Y-I080V-N085S-E087D-S099R-T114Q-S126A-D127E-F128G-N242D | 45 | 2.0 | 2.4 | 2.8 |
| WALBSP-07069 (comparative) | A037T-S039E-I043V-A047V-T056Y-I080V-N085S-E087D-T114Q-S126A-D127E-F128G-N242D | 46 | 0.8 | 0.3 | 1.0 |
| WALBSP-07044 (comparative) | A037T-S039E-A047V-T056Y-I080V-N085S-E087D-S099R-S126A-D127E-F128G-N242D | 46 | 2.1 | 2.1 | 2.7 |
| WALBSP-07100 (comparative) | S039E-S099R-S126A-D127E-F128G-Q256E | 47 | 2.3 | 3.0 | 3.8 |
| WALBSP-07013 (comparative) | S039E-S099R-S126A-D127E-F128G-N242D | 47 | 2.1 | 2.2 | 2.6 |
| WALBSP-07139 (comparative) | S039E-S099R-S126A-D127E-F128G-Q200L | 48 | 2.2 | 2.8 | 3.7 |
| WALBSP-07077 (comparative) | A037T-S039E-I043V-A047V-P054T-T056Y-S099R-T114Q-S126A-D127E-F128G-N242D | 51 | 2.2 | 2.8 | 3.6 |
| WALBSP-07087 (comparative) | A037T-S039E-I043V-A047V-P054T-T056Y-S099R-S126A-D127E-F128G-N242D | 51 | 2.1 | 2.8 | 3.6 |
| WALBSP-07138 (comparative) | S039E-N074D-I080V-S099R-S126A-D127E-F128G | 53 | 2.3 | 2.8 | 3.8 |
| WALBSP-07021 (comparative) | A037T-S039E-I043V-A047V-P054T-T056Y-N085S-E087D-S099R-S126A-D127E-F128G-N242D | 54 | 1.9 | 2.4 | 2.9 |
| WALBSP-07039 (comparative) | A037T-S039E-I043V-A047V-T056Y-N085S-E087D-S099R-T114Q-S126A-D127E-F128G-N242D | 54 | 2.0 | 2.5 | 3.0 |
| WALBSP-07053 (comparative) | A037T-S039E-I043V-A047V | 54 | 0.8 | 0.8 | 0.7 |
| WALBSP-07073 (comparative) | A037T-S039E-I043V-A047V-N242D | 54 | 0.7 | 0.6 | 0.9 |
| WALBSP-07118 (comparative) | S039E-P054T-N074D-S099R-S126A-D127E-F128G | 55 | 2.3 | 2.8 | 4.2 |
| WALBSP-07108 (comparative) | S039E-A047V-N074D-S099R-S126A-D127E-F128G | 55 | 2.3 | 2.7 | 3.3 |
| WALBSP-07057 (comparative) | A037T-S039E-I043V-A047V-P054T-T056Y-N085S-E087D-S099R-T114Q-S126A-D127E-F128G-N242D | 55 | 2.0 | 2.3 | 2.7 |
| WALBSP-07089 (comparative) | S039E-N074D-S099R-T114Q-S126A-D127E-F128G | 55 | 2.5 | 2.8 | 3.8 |
| WALBSP-07148 (comparative) | S039E-N074D-N085S-S099R-S126A-D127E-F128G | 56 | 2.3 | 3.0 | 3.4 |
| WALBSP-07074 (comparative) | A037T-S039E-I043V-A047V-T056Y-N085S-E087D-S099R-S126A-D127E-F128G-N242D | 57 | 1.8 | 2.1 | 2.5 |
| WALBSP-07079 (comparative) | S039E-N074D-E087D-S099R-S126A-D127E-F128G | 57 | 2.3 | 3.4 | ND |
| WALBSP-07098 (comparative) | S039E-I043V-N074D-S099R-S126A-D127E-F128G | 57 | ND | 2.7 | 3.5 |
| WALBSP-07128 (comparative) | S039E-T056Y-N074D-S099R-S126A-D127E-F128G | 58 | 2.2 | 3.0 | 3.6 |
| WALBSP-07099 (comparative) | S039E-N074D-S099R-S126A-D127E-F128G-N242D | 77 | 2.2 | 3.0 | 3.8 |

As it can be seen from Table 2 the variants of the composition of the invention are considerable better than the parent protease for the removal of egg stains

## Claims

1. A phosphate-free automatic dishwashing cleaning composition comprising:
i) a protease wherein the protease is a variant having at least 90% identity with the amino acid sequence of SEQ ID NO:1 and comprising the following substitutions: P54T, S126A, D127E and F128G (using the SEQ ID NO: 1 numbering); and
ii) from 10 to 50% by weight of the composition of a complexing agent system comprising from 0 to less than 30% by weight of the composition of citric acid.

2. A composition according claim 1 wherein the complexing agent system comprises a complexing agent selected from the group consisting of methyl glycine diacetic acid, glutamic-N,N-diacetic acid, iminodisuccinic acid, carboxy methyl inulin and mixtures thereof, preferably a salt of methyl glycine diacetic acid and wherein the complexing agent system preferably comprises citric acid and methyl glycine diacetic acid preferably in a weight ratio of from about 0.5:1 to about 2:1.

3. A composition according to any of the preceding claims wherein the composition comprises a bleaching system wherein the composition comprises at least 10% by weight thereof of bleach and preferably a bleach activator or a bleach catalyst.

4. A composition according to any of the preceding claims comprising:
i) a bleaching system comprising at least 10% by weight of the composition of percarbonate and optionally a bleach activator and/or a bleach catalyst;
ii) a non-ionic surfactant;
iii) a dispersant polymer;
iv) an amylase; and
v) optionally but preferably a glass care agent.

5. A method of washing soiled dishware in a dishwasher using soft water comprising the steps of:
i) providing the soiled dishware;
ii) treating the dishware with a cleaning composition according to any of the preceding claims; and
iii) optionally rinsing the dishware.

## Patentansprüche

1. Phosphatfreie Maschinen-Geschirrspülreinigungszusammensetzung, umfassend:
i) eine Protease, wobei die Protease eine Variante ist, die zu wenigstens 90 % mit der Aminosäuresequenz von SEQ ID NO: 1 identisch ist und die folgenden Substitutionen umfasst: P54T, S126A, D127E und F128G (unter Verwendung der SEQ ID NO: 1 Nummerierung); und
ii) zu 10 bis 50 Gew.-% der Zusammensetzung ein Komplexbildnersystem, umfassend zu 0 bis weniger als 30 Gew.-% der Zusammensetzung Citronensäure.

2. Zusammensetzung nach Anspruch 1, wobei das Komplexbildnersystem einen Komplexbildner, der ausgewählt ist aus der Gruppe bestehend aus Methylglycindiessigsäure, Glutamin-N,N-diessigsäure, Iminodibernsteinsäure, Carboxymethylinulin und Mischungen davon, vorzugsweise einem Salz von Methylglycindiessigsäure, umfasst und wobei das Komplexbildnersystem vorzugsweise Zitronensäure und Methylglycindiessigsäure, vorzugsweise in einem Gewichtsverhältnis von etwa 0,5 : 1 bis etwa 2 : 1, umfasst.

3. Zusammensetzung nach einem der vorstehenden Ansprüche, wobei die Zusammensetzung ein Bleichsystem umfasst, wobei die Zusammensetzung wenigstens zu 10 Gew.-% davon Bleichmittel und vorzugsweise einen Bleichaktivator oder einen Bleichkatalysator umfasst.

4. Zusammensetzung nach einem der vorstehenden Ansprüche, umfassend:
i) ein Bleichsystem, umfassend wenigstens zu 10 Gew.-% der Zusammensetzung Percarbonat und wahlweise einen Bleichaktivator und/oder einen Bleichkatalysator;
ii) ein nichtionisches Tensid;
iii) ein Dispergierpolymer;
iv) eine Amylase; und
v) wahlweise, jedoch vorzugsweise, ein Glaspflegemittel.

5. Verfahren zum Waschen von verschmutztem Geschirr in einem Geschirrspüler unter Verwendung von Weichwasser, umfassend die Schritte:
i) Bereitstellen des verschmutzten Geschirrs;
ii) Behandeln des Geschirrs mit einer Reinigungszusammensetzung nach einem der vorstehenden Ansprüche; und
iii) wahlweise Abspülen des Geschirrs.

## Revendications

1. Composition de nettoyage sans phosphate pour le lavage automatique de la vaisselle comprenant :
i) une protéase, dans laquelle la protéase est un variant ayant au moins 90 % d'identité avec la séquence d'acides aminés de SEQ ID NO:1 et comprenant les substitutions suivantes : P54T, S126A, D127E et F128G (à l'aide de la numérotation de SEQ ID NO:1) ; et
ii) de 10 à 50 % en poids de la composition d'un système d'agent complexant comprenant de 0 à moins de 30 % en poids de la composition d'acide citrique.

2. Composition selon la revendication 1, dans laquelle le système d'agent complexant comprend un agent complexant choisi dans le groupe constitué d'acide méthylglycine diacétique, acide glutamique-N,N-diacétique, acide iminodisuccinique, carboxy méthyl inuline et des mélanges de ceux-ci, de préférence un sel d'acide méthylglycine diacétique et dans laquelle le système d'agent complexant comprend de préférence de l'acide citrique et de l'acide méthylglycine diacétique, de préférence dans un rapport en poids d'environ 0,5:1 à environ 2:1.

3. Composition selon l'une quelconque des revendications précédentes, dans laquelle la composition comprend un système de blanchiment dans laquelle la composition comprend au moins 10 % en poids d'agent de blanchiment et, de préférence, d'un activateur de blanchiment ou d'un catalyseur de blanchiment.

4. Composition selon l'une quelconque des revendications précédentes comprenant :
i) un système de blanchiment comprenant au moins 10 % en poids de la composition de percarbonate et éventuellement d'un activateur de blanchiment et/ou d'un catalyseur de blanchiment ;
ii) un agent tensioactif non ionique ;
iii) un polymère dispersant ;
iv) une amylase ; et
v) éventuellement, mais de préférence, un agent d'entretien du verre.

5. Procédé de lavage de vaisselle souillée dans un lave vaisselle à l'aide d'eau douce comprenant les étapes consistant à :
i) fournir la vaisselle souillée ;
ii) traiter la vaisselle avec une composition de nettoyage selon l'une quelconque des revendication précédentes ; et
iii) éventuellement rincer la vaisselle.
